(19) Europäisches Patentamt
European Patent Office
Office européen des brevets

(11) Veröffentlichungsnummer: **0 284 969 B1**

(12) # EUROPÄISCHE PATENTSCHRIFT

(45) Veröffentlichungstag der Patentschrift: **28.11.90**

(51) Int. Cl.$^5$: **C07D 309/08**

(21) Anmeldenummer: **88104601.5**

(22) Anmeldetag: **23.03.88**

(54) **Verfahren zur Herstellung von Tetrahydropyran-4-carbonsaeureestern.**

(30) Priorität: **31.03.87 DE 3710805**

(43) Veröffentlichungstag der Anmeldung:
**05.10.88 Patentblatt 88/40**

(45) Bekanntmachung des Hinweises auf die Patenterteilung:
**28.11.90 Patentblatt 90/48**

(84) Benannte Vertragsstaaten:
**BE CH DE FR GB IT LI**

(56) Entgegenhaltungen:
**BEILSTEINS HANDBUCH DER ORGANISCHEN CHEMIE; 4. Auflage, Band 18, Teil 5, 1976, Springer-Verlag, Berlin; * Seite 3836 *; & JOURNAL OF THE CHEMICAL SOCIETY, 1930, Seiten 2525-2530 (Kat. D)**
**HOUBEN-WEYL: "Methoden der organischen Chemie", 4. Auflage, Band VI, Teil 4, 1966, Georg Thieme Verlag, Stuttgart; * Seite 25 ***
**JOURNAL OF ORGANIC CHEMISTRY, Band 47, Nr, 12, 1982, Seiten 2364-2369; C.K. Lai et al.: "Total synthesis of racemic triptolide and triptonide", *S. 2366, Spalte 1, Herstellung von Verbindung 8 * .**

(73) Patentinhaber: **BASF Aktiengesellschaft, Carl-Bosch-Strasse 38, D-6700 Ludwigshafen(DE)**

(72) Erfinder: **Becker, Rainer, Dr., Im Haseneck 22, D-6702 Bad Duerkheim(DE)**
Erfinder: **Eckhardt, Heinz, Dr., Ruedigerstrasse 7, D-6700 Ludwigshafen(DE)**
Erfinder: **Fischer, Rolf, Dr., Bergstrasse 98, D-6900 Heidelberg(DE)**
Erfinder: **Spiegler, Wolfgang, Dr., Westpreussentrasse 5, D-6520 Worms 27(DE)**
Erfinder: **Vagt, Uwe, Dr., Paul-Neumann-Strasse 44, D-6720 Speyer(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Diese Erfindung betrifft ein Verfahren zur Herstellung von Tetrahydropyran-4-carbonsäureestern durch Umsetzung von 3-Hydroxyethylbutyrolacton oder dessen Estern und Ethern mit Alkoholen in Gegenwart von sauer wirkenden Katalysatoren.

Tetrahydropyran-4-carbonsäureester kann man nach den Angaben in Journ. Chem. Soc. 1930, Seiten 2525 - 2530 dadurch herstellen, daß man β, β'-Dichlordiethylether mit dem Natriumsalz des Malonsäurediethylesters zu Tetrahydropyran-4.4-dicarbonsäurediethylester umsetzt, daraus durch Hydrolyse Tetrahydropyran-4.4-dicarbonsäure gewinnt, die man in Tetrahydropyran-4-carbonsäure überführt, welche man schließlich verestert.

Es bestand die Aufgabe, ein einfacheres, wirtschaftliches Verfahren zur Herstellung von Tetrahydropyran-4-carbonsäureestern zu entwickeln.

Diese Aufgabe wird durch das erfindungsgemäße Verfahren gelöst. Danach kann man Tetrahydropyran-4-carbonsäureester der Formel

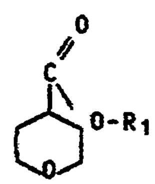

I,

in der $R_1$ einen Alkylrest mit 1 bis 6 Kohlenstoffatomen, einen Cycloalkylrest mit 5 bis 7 Kohlenstoffatomen oder einen Arylrest mit 6 bis 10 Kohlenstoffatomen bedeutet, auf besonders vorteilhafte Weise dadurch herstellen, daß man Butyrolactone der Formel

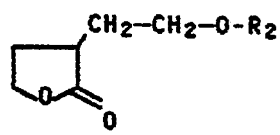

II,

in der $R_2$ ein Wasserstoffatom, eine Alkylgruppe mit 1 bis 6 Kohlenstoffatomen oder eine Acylgruppe der Formel -CO-$R_3$ und $R_3$ ein Wasserstoffatom oder eine Alkylgruppe mit 1 bis 6 Kohlenstoffatomen bedeuten, mit Alkoholen der Formel

$R_1$-OH III,

in der $R_1$ die oben genannte Bedeutung hat, bei Temperaturen von 150 bis 400°C in Gegenwart von sauren Katalysatoren umsetzt.

Die Bildung von Tetrahydropyran-4-carbonsäureestern aus 3-Hydroxyethylbutyrolacton bzw. dessen Derivaten und Alkoholen mit guten Selektivitäten war nicht vorherzusehen, da zahlreiche, zu anderen Produkten führende Konkurrenzreaktionen hätten ablaufen können.

Die erfindungsgemäße Umsetzung läßt sich für den Fall der Herstellung von Tetrahydropyran-4-carbonsäuremethylester aus 3-Hydroxyethylbutyrolacton und Methanol durch die folgende Formelgleichung wiedergeben:

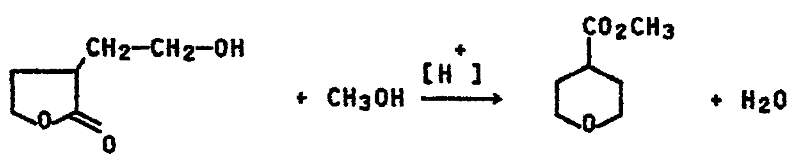


Geeignete Lactone der Formel II sind z. B. 3-Hydroxyethylbutyrolacton, dessen Hydroxyethylgruppe z. B. zum Methyl-, Ethyl-, Propyl-, Butyl-ether verethert oder z. B. mit Ameisensäure, Essigsäure, Propionsäure, Buttersäure-, n- und i-Valeriansäure verestert sein kann. Geeignete Alkohole der Formel III sind beispielsweise Methanol, Ethanol, n-Propanol, i-Propanol, tert.-Butanol, n-Butanol, i-Butanol, sec.-Butanol, n-Pentanol, n-Hexanol, Phenol, Cyclopentanol, Cyclohexanol. Besonders geeignet sind Methanol, Ethanol und Propanole.

Das Molverhältnis von Butyrolacton II zu Alkohol III beträgt vorteilhaft 1 : 0,5 bis 1 : 10, insbesondere 1 : 1 bis 1 : 5. Die Umsetzung führt man bei einer Temperatur von 150 bis 400°C, vorzugsweise bei 200 bis 400°C, insbesondere bei 200 bis 300°C durch. Vorteilhaft wählt man Drücke von 1 bis 100 bar, insbesondere von 1 bis 10 bar.

Geeignete saure Katalysatoren sind z. B. sauer wirkende Oxide von Elementen der III. und IV. Hauptgruppe sowie der IV. bis VI. Nebengruppe des Periodischen Systems, die gegenüber Protonen- oder Lewis-Sauren bevorzugt sind. Beispielsweise seien heterogene saure Katalysatoren genannt, wie Siliciumdioxid in Form von Kieselgel, Kieselgur, Quarz, weiterhin Titandioxid, Zirkondioxid, Phosphorpentoxid, Vanadiumpentoxid, Bortrioxid, Aluminiumoxid, Chromoxide, Molybdänoxide, Wolframoxide oder deren Gemische. Es sind auch Zeolithe, wie z.B. Y-Zeolithe geeignet.

Zweckmäßigerweise setzt man die Butyrolactone II an derartigen Festbettkatalysatoren in der Gasphase um. Man arbeitet dabei vorteilhaft mit einer Katalysatorbelastung von 0,1 bis 10, insbesondere von 0,1 bis 5 g Butyrolacton II pro g Katalysator und Stunde.

Die Umsetzung kann diskontinuierlich oder kontinuierlich als Festbettreaktion mit Festbettkatalysatoren, beispielsweise nach der Sumpf- oder Rieselfahrweise in der Flüssig- oder Gasphase, z. B. im Wirbelbett oder aber mit in der Flüssigphase suspendierten Festbettkatalysatoren durchgeführt werden. Man kann aber auch in der Flüssigphase homogen gelöste saure Katalysatoren einsetzen. Als solche sauren Katalysatoren kommen z. B. Mineralsäuren, wie Schwefelsäure, Phosphorsäure, Salzsäure, Salpetersäure, Bromwasserstoffsäure oder Sulfonsäuren, wie Benzolsulfonsäure oder p-Toluolsulfonsäure in Betracht. Das Molverhältnis von Butyrolacton II zur Säure beträgt beispielsweise 1 : 0,001 bis 1 : 1, insbesondere 1 : 0,01 bis 1 : 0,1.

Die Umsetzung der Butyrolactone mit den Alkoholen in der Flüssigphase wird beispielsweise so durchgeführt, daß man ein Gemisch aus dem Lacton und dem jeweiligen Alkohol in Gegenwart eines suspendierten Festbettkatalysators oder eines homogen gelösten Katalysators auf die gewünschte Reaktionstemperatur erhitzt. Nach Ablauf der notwendigen Reaktionszeit wird das Reaktionsgemisch abgekühlt und der saure Katalysator, z. B. durch Filtration oder Neutralisation, entfernt. Das Reaktionsgemisch wird anschließend zur Gewinnung der gewünschten Tetrahydropyran-4-carbonsäureester fraktionierend destilliert.

Nach einer bevorzugten Ausführungsform des erfindungsgemäßen Verfahrens in der Gasphase wird zunächst ein Gemisch aus dem Butyrolacton und dem jeweiligen Alkohol verdampft und dann, gegebenenfalls zusammen mit einem Inertgas wie Stickstoff, Kohlendioxid oder Argon, bei der gewünschten Reaktionstemperatur gasförmig über einen fest angeordneten Katalysator geleitet, wobei der Katalysator vorteilhaft in auf- und abwirbelnder Bewegung gehalten wird. Der Reaktionsaustrag wird mittels geeigneter Kühlvorrichtung kondensiert und anschließend durch fraktionierende Destillation aufgearbeitet. Der gewünschte Tetrahydropyran-4-carbonsäureester wird abgetrennt. Nicht umgesetztes Hydroxyethylbutyrolacton bzw. dessen Ether oder Ester können in die Reaktion zurückgeführt werden.

3-Hydroxyethylbutyrolacton kann z. B. durch Umsetzung von Acetessigester mit Ethylenoxid hergestellt werden.

Die nach dem erfindungsgemäßen Verfahren erhältlichen Tetrahydropyran-4-carbonsäureester stellen wertvolle Zwischenprodukte dar, die z. B. zu dem gesuchten Tetrahydropyran-4-aldehyd weiterverarbeitet werden können.

Beispiel 1

a) Pro Stunde wurden 100 ml einer Lösung, die aus 52 Gew.% 3-Hydroxyethylbutyrolacton, 38 Gew.% Methanol und 10 Gew.% Wasser bestand, in einen Verdampfer (300°C) gepumpt und von dort mit 175 l Stickstoff über 300 g (350 ml) Aluminiumoxid-Wirbelkontakt (Korngröße 0,1 -0,3 mm) bei einer Reaktionstemperatur von 270°C geleitet. Die Versuchsdauer betrug 12,5 h, der Gesamteinsatz: 650 g (5,0 mol) Hydroxyethylbutyrolacton. Der gasförmige Reaktionsaustrag wurde in Kühlfallen kondensiert und im Vakuum fraktionierend destilliert. Dabei wurden die folgenden drei Fraktionen in den angegebenen Ausbeuten erhalten.

F1 260 g (36 % d.Th.) Tetrahydropyran-4-carbonsäuremethylether
(Sdp. 86-90°C/24 mbar)
F2 162 g (29 % d.Th.) 3-Vinylbutyrolacton
(Sdp. 105-109°C/24 mbar)
F3 144 g (20 % d.Th.) 3-Methoxyethylbutyrolacton
(Sdp. 137°C/24 mbar)

Die Fraktionen 2 und 3 wurden wie folgt beschrieben erneut eingesetzt.

b) Pro Stunde wurden 100 ml einer Lösung, die aus 52 Gew.% 3-Methoxyethylbutyrolacton, 38 Gew.% Methanol und 10 Gew.% Wasser bestand, in einen Verdampfer (300°C) gepumpt und von dort mit 175 l Stickstoff über 300 g (350 ml) Aluminiumoxid-Wirbelkontakt (Korngröße 0,1 -0,3 mm) bei einer Reaktionstemperatur von 270°C geleitet. Die Versuchsdauer betrug 3 h, der Gesamteinsatz: 144 g (1,0 mol) 3-Methoxyethylbutyrolacton aus Fraktion F3. Der gasförmige Reaktionsaustrag wurde in Kühlfallen kondensiert und im Vakuum fraktionierend destilliert. Dabei wurden die folgenden drei Fraktionen in den angegebenen Ausbeuten erhalten:

F1 37 g (26 % d.Th.) Tetrahydropyran-4-carbonsäuremethylester
(Sdp. 86-90°C/24 mbar)
F2 34 g (30 % d.Th.) 3-Vinylbutyrolacton
(Sdp. 105-109°C/24 mbar)

F3 45 g (31 % d.Th.) 3-Methoxyethylbutyrolacton
(Sdp. 137°C/24 mbar)

c) Pro Stunde wurden 100 ml einer Lösung, die aus 52 Gew.% 3-Vinylbutyrolacton, 38 Gew.% Methanol und 10 Gew.% Wasser bestand, in einen Verdampfer (300°C) gepumpt und von dort mit 175 l Stickstoff über 300 g (350 ml) Aluminiumoxid-Wirbelkontakt (Korngröße 0,1 - 0,3 mm) bei einer Reaktionstemperatur von 270°C geleitet. Die Versuchsdauer betrug 3 h, der Gesamteinsatz: 162 g (1,45 mol) 3-Vinylbutyrolacton aus Fraktion F2. Der gasförmige Reaktionsaustrag wurde in Kühlfallen kondensiert und im Vakuum fraktionierend destilliert. Dabei wurden die folgenden drei Fraktionen in den angegebenen Ausbeuten erhalten:

F1 21 g (10 % d.Th.) Tetrahydropyran-4-carbonsäuremethylester
(Sdp. 86-90°C/24 mbar)
F2 112 g (69 % d.Th.) 3-Vinylbutyrolacton
(Sdp. 105-109°C/24 mbar)
F3 17 g (8 % d.Th.) 3-Methoxyethylbutyrolacton
(Sdp. 137°C/24 mbar)

Die Gesamtausbeute (a + b + c) betrug somit:

F1 318 g (44 % d.Th.) Tetrahydropyran-4-carbonsäuremethylester
(Sdp. 86-90°C/24 mbar)
F2 146 g (29 % d.Th.) 3-Vinylbutyrolacton
(Sdp. 105-109°C/24 mbar)
F3 62 g (20 % d.Th.) 3-Methoxyethylbutyrolacton
(Sdp. 137°C/24 mbar)

Beispiel 2 (Temperaturabhängigkeit)

Pro Stunde wurden 100 ml einer Lösung, die aus 52 Gew.% 3-Hydroxyethylbutyrolacton, 38 Gew.% Methanol und 10 Gew.% Wasser bestand, in einen Verdampfer (300°C) gepumpt und von dort mit 175 l Stickstoff über 300 g (350 ml) Aluminiumoxid-Wirbelkontakt (Korngröße 0,1 -0,3 mm) bei den in der folgenden Tabelle angegebenen Reaktionstemperaturen geleitet. Die Versuchsdauer betrug jeweils 4 h. Der gasförmige Reaktionsaustrag wurde in Kühlfallen kondensiert und gaschromatografisch analysiert. Dabei wurden die folgenden Ergebnisse erhalten:

| Temperatur ($^0C$) | Tetrahydropyran-4-carbonsäuremethylester (Mol-%) | 3-Vinylbutyrolacton (Mol-%) | 3-Methoxyethylbutyrolacton (Mol-%) | 3-Hydroxyethylbutyrolacton (Mol-%) |
|---|---|---|---|---|
| 300 | 14 | 56 | 12 | < 1 |
| 270 | 36 | 27 | 20 | 4 |
| 260 | 41 | 21 | 20 | 5 |
| 250 | 40 | 16 | 18 | 10 |
| 240 | 42 | 16 | 20 | 10 |

Beispiel 3

Pro Stunde wurden 150 ml einer Lösung, die aus 52 Gew.% 3-Hydroxyethylbutyrolacton, 38 Gew.% Methanol und 10 Gew.% Wasser bestand, in einen Verdampfer gempumpt und von dort mit 175 l Stickstoff über 300 g (350 ml) Aluminiumoxid-Wirbelkontakt (Korngröße 0,1 bis 0,3 mm) bei einer Reaktionstemperatur von 260°C geleitet. Die Versuchsdauer betrug 17 h, der Gesamteinsatz: 1300 g (10,0 mol) 3-Hydroxyethyl-butyrolacton.

Der gasförmige Reaktionsaustrag wurde in Kühlfallen kondensiert und im Vakuum fraktionierend destilliert. Dabei wurde nach Abtrennung von Methanol und Wasser als erste Fraktion reiner Tetrahydropyran-4-carbonsäuremethylester (Sdp. 90 bis 93°C/30 mbar) erhalten.

Die noch in der Destillationsblase verbliebene Substanz wurde anschließend im Vakuum destilliert und so mit Methanol und Wasser versetzt, daß wiederum eine 52 gew.%ige Lsung in Methanol/Wasser entstand. Diese wurde wie oben beschrieben erneut in die Reaktion eingesetzt. Dieser Vorgang wurde insgesamt noch dreimal wiederholt.

Gesamtausbeute nach Destillation: 791 g (55 % d.Th) Tetrahydropyran-4-carbonsäuremethylester

**Patentansprüche**

1. Verfahren zur Herstellung von Tetrahydropyran-4-carbonsäureestern der Formel

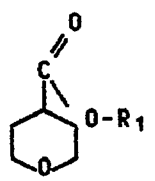

I,

in der $R_1$ einen Alkylrest mit 1 bis 6 Kohlenstoffatomen, einen Cycloalkylrest mit 5 bis 7 Kohlenstoffatomen oder einen Arylrest mit 6 bis 10 Kohlenstoffatomen bedeutet, dadurch gekennzeichnet, daß man Butyrolactone der Formel

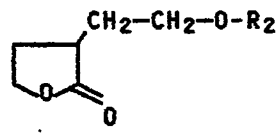

II,

in der $R_2$ ein Wasserstoffatom, eine Alkylgruppe mit 1 bis 6 Kohlenstoffatomen oder eine Acylgruppe der Formel $-CO-R_3$, und $R_3$ ein Wasserstoffatom oder eine Alkylgruppe mit 1 bis 6 Kohlenstoffatomen bedeuten, mit Alkoholen der Formel

$R_1$-OH III,

in der $R_1$ die oben genannte Bedeutung hat, bei Temperaturen von 150 bis 400°C in Gegenwart von sauren Katalysatoren umsetzt.

2. Verfahren nach Anspruch 1, dadurch gekennzeichnet, daß man die Umsetzung bei 200 bis 300°C vornimmt.

3. Verfahren nach Anspruch 1, dadurch gekennzeichnet, daß man als Katalysatoren sauer wirkende Oxide von Elementen der III. und IV. Hauptgruppe sowie der IV. bis VI. Nebengruppe verwendet.

4. Verfahren nach Anspruch 1, dadurch gekennzeichnet, daß man als Katalysatoren Aluminiumoxid, Siliciumdioxid, Titandioxid, Zirkondioxid, Phosphorpentoxid, Vanadinpentoxid, Bortrioxid oder Oxide des Chroms, Molybdäns oder Wolframs verwendet.

5. Verfahren nach Anspruch 1, dadurch gekennzeichnet, daß man die Umsetzung der Butyrolactone mit den Alkoholen in Gegenwart der Katalysatoren in der Gas- oder Flüssigphase vornimmt.

**Revendications**

1. Procédé de préparation d'esters d'acide tétrahydropyranne-4-carboxylique de formule

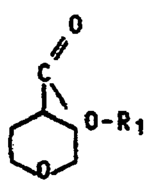

I,

dans laquelle $R_1$ est un reste alkyle ayant de 1 à 6 atomes de carbone, un reste cycloalkyle ayant de 5 à 7 atomes de carbone ou un reste aryle ayant de 6 à 10 atomes de carbone, caractérisé en ce qu'on fair réagir une butyrolactone de formule

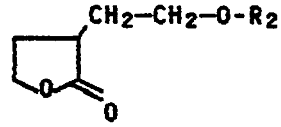

II,

dans laquelle $R_2$ est un atome d'hydrogène, un groupement alkyle ayant de 1 à 6 atomes de carbone ou un groupement acyle de formule $-CO-R_3$ et $R_3$ est un atome d'hydrogène ou un groupement alkyle ayant de 1 à 6 atomes de carbone, avec des alcools de formule

$R_1$-OH III.

dans laquelle $R_1$ a la signification donnée précédemment, à des températures de 150° à 400°C en présence de catalyseurs acides.

2. Procédé selon la revendication 1, caractérisé en ce qu'on mène la réaction à 200–300°C.

3. Procédé selon la revendication 1, caractérisé en ce qu'on utilise comme catalyseurs des oxydes à action acide des éléments des groupes IIIa et IVa ainsi que des groupes IVb à VIb.

4. Procédé selon la revendication 1, caractérisé en ce qu'on utilise comme catalyseurs de l'oxyde d'aluminium, du dioxyde de silicium, du dioxyde de titane, du dioxyde de zirconium, du pentoxyde de phosphore, du pentoxyde de vanadium, du rinoxyde de bore ou des oxydes de chrome, de molybdène ou de tungstène.

5. Procédé selon la revendication 1, caractérisé en ce qu'on mène la réaction de la butyrolactone avec l'alcool en présence de catalyseurs, en phase gazeuse ou liquide.

**Claims**

1. A process for preparing a tetrahydropyran-4-carboxylic ester of the formula

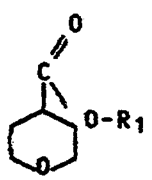

I,

where $R_1$ is alkyl of from 1 to 6 carbon atoms, cycloalkyl of from 5 to 7 carbon atoms or aryl of from 6 to 10 carbon atoms, which comprises reacting a butyrolactone of the formula

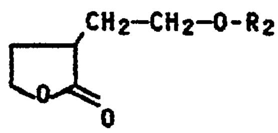

II,

where $R_2$ is hydrogen, alkyl of from 1 to 6 carbon atoms or acyl of the formula CO–$R_3$, and $R_3$ is hydrogen or alkyl of from 1 to 6 carbon atoms, with an alcohol of the formula

$R_1$–OH III

where $R_1$ is as defined above, at 150–400°C in the presence of an acidic catalyst.

2. A process as claimed in claim 1, wherein the reaction is carried out at 200–300°C.

3. A process as claimed in claim 1, wherein the catalyst used is an acidic oxide of an element of main group III or IV or of subgroup IV, V or VI.

4. A process as claimed in claim 1, wherein the catalyst used is aluminum oxide, silicon dioxide, titanium dioxide, zirconium dioxide, phosphorus pentoxide, vanadium pentoxide, boron trioxide or an oxide of chromium, of molybdenum of of tungsten.

5. A process as claimed in claim 1, wherein the reaction of butyrolactone with an alcohol in the presence of a catalyst is carried out in the gas or liquid phase.